# EUROPEAN PATENT APPLICATION

(11) **EP 3 903 665 A1**
(43) Date of publication of application: **03.11.2021**
(21) Application number: 19902670.9
(22) Date of filing: 04.12.2019
(51) Int. Cl.: A61B 5/00

(54) **SAMPLE COLLECTION TOOL FOR ANIMALS**

(30) Priority: 27.12.2018 KR 20180170396
(71) Applicant: Noblebio Co., Ltd., Hwaseoug-si, Gyeonggi-do 18521 (KR)
(72) Inventor: BAEK, Kye Seung, Suwon-si Gyeonggi-do 16383 (KR)
(74) Representative: Gulde & Partner
(86) International application number: PCT/KR2019/016971
(87) International publication number: WO 2020/138746

(57) **Abstract**

Provided is an animal specimen collection tool, and more particularly to, an animal specimen collection tool in which a collection portion is formed of a silicone or urethane material that is not easily torn in a soft material, when the animal bites and chews the collection portion directly in the mouth of the animal, the saliva of animal is absorbed in a sponge of a hydrophilic material provided inside through an inlet hole of the collection portion, the collection portion is removed from the mouth of the animal, and then inserted into the inside a tube portion and pressed to a tube adapter, and the saliva of animal absorbed in the sponge is discharged through the inlet hole of the collection portion and stored in the tube portion, which prevent contamination of the specimen, and thus DNA/RNA/antibody, etc. may be safely and accurately extracted, and the collection method and specimen storage are simple and convenient, which increase efficiency.

## Description

### TECHNICAL FIELD

The present invention relates to an animal specimen collection tool, and more particularly to, an animal specimen collection tool in which a collection portion is formed of a silicone or urethane material that is not easily torn in a soft material, when the animal bites and chews the collection portion directly in the mouth of the animal, the saliva of animal is absorbed in a sponge of a hydrophilic material provided inside through an inlet hole of the collection portion, the collection portion is removed from the mouth of the animal, and then inserted into the inside a tube portion and compressed to a tube adapter, and the saliva of animal absorbed in the sponge is discharged through the inlet hole of the collection portion and stored in the tube portion, which prevent contamination of the specimen, and thus DNA/RNA/antibody, etc. may be safely and accurately extracted, and the collection method and specimen storage are simple and convenient, which increase efficiency.

### BACKGROUND ART

Saliva collection for diagnosing and analyzing a specimen is performed in several ways.

There are a method of collecting saliva using a general swab (a small sample) and a method of spitting and collecting saliva using a saliva collection kit (a large sample). Most methods are used for the human body, but are used for the human body except for granulation, etc.

Here, the method of collection saliva in granulation, etc. uses a general swab or a saliva-only sponge.

However, as in Korean Laid-Open Patent Publication No. 10-2014-0005535, there are cases in which the method used for the human body is not used for animals.

Among them, saliva collection for pig disease diagnosis is subject to various restrictions. Pigs have a habit of biting when they look at objects, so it is impossible to collect saliva with a general collection stick.

The currently used method is to collect saliva from a silk thread by hand after the pig bites the silk thread by hanging the silk thread.

Therefore, when the silk thread is hung, several pigs may bite the silk thread and it may also cause serious contamination of the sample. Such contamination is subject to several limitations when extracting DNA/RNA/antibody.

### DESCRIPTION OF EMBODIMENTS

### TECHNICAL PROBLEM

Provided is an animal specimen collection tool in which a collection portion is formed of a silicone or urethane material that is not easily torn in a soft material,

When the animal bites and chews the collection portion directly in the mouth of the animal, the saliva of animal is absorbed in a sponge of a hydrophilic material provided inside through an inlet hole of the collection portion, the collection portion is removed from the mouth of the animal, and then inserted into the inside a tube portion and compressed to a tube adapter, and the saliva of animal absorbed in the sponge is discharged through the inlet hole of the collection portion and stored in the tube portion, which prevent contamination of the specimen, and thus DNA/RNA/antibody, etc. may be safely and accurately extracted, and the collection method and specimen storage are simple and convenient, which increase efficiency.

### SOLUTION TO PROBLEM

According to an aspect of the present invention, an animal specimen collection tool includes a collection portion in direct contact with a mouth of animal, having a hollow inside to form a plurality of input holes in an outer surface such that saliva of the animal is introduced, one side opened to be communicatively connected to the hollow inside, and a connection hole formed in the opened outer periphery, and formed of a soft, flexible and elastic material;

A sponge formed in the hollow inside of the collection portion, absorbing the saliva of the animal introduced through the input hole, and formed of a hydrophilic material;

A connection adapter having a connection protrusion protruding so as to be connected to the connection hole of the collection portion, and connecting the collection portion and a collection stick;

The collection stick having the connection adapter connected to the collection portion inserted into and fixed to inside of one side;

And a tube portion into which the collection portion is inserted compressing the collection portion and the sponge to discharge the saliva of the animal absorbed by the sponge to the outside through the input hole of the collection portion, and storing the saliva of the animal discharged from the collection portion therein.

The collection portion may be formed in the shape of a corrugated tube so as to be easily compressed inside the tube portion.

The connection adapter may have the connection protrusion protruding from both sides of the outer periphery to be formed in a "T" shape, is inserted through the opened one side of the collection portion to couple the connection protrusion to the connection hole inside the collection portion, and then is inserted into and fixed to the inside of one side of the collection stick.

A fixing cover may be installed on one side of the collection stick such that the connection adapter connected to the collection portion is inserted and then fixed, and a through hole is formed in a central portion of the fixing cover such that the collection portion connected to the connection adapter protrudes to the outside.

A tube adapter in direct contact with the collection portion input into the tube portion to compress the collection portion and the sponge may be further formed inside the tube portion, and one side surface of the tube adapter may be formed in a mesh network structure such that the saliva of the animal discharged by the compression of the collection portion and the sponge penetrates and is stored in the tube portion.

### ADVANTAGEOUS EFFECTS OF DISCLOSURE

As described above, the animal specimen collection tool of the present invention in which a collection portion is formed of a silicone or urethane material that is not easily torn in a soft material, when the animal bites and chews the collection portion directly in the mouth of the animal, the saliva of animal is absorbed in a sponge of a hydrophilic material provided inside through an inlet hole of the collection portion, the collection portion is removed from the mouth of the animal, and then inserted into the inside a tube portion and compressed to a tube adapter, and the saliva of animal absorbed in the sponge is discharged through the inlet hole of the collection portion and stored in the tube portion may prevent contamination of the specimen, thereby safely and accurately extracting DNA/RNA/antibody, etc., and may have the simple and convenient collection method and specimen storage, thereby increasing efficiency.

### BRIEF DESCRIPTION OF DRAWINGS

FIG. 1 is an exploded perspective view showing an animal specimen collection tool according to an embodiment of the present invention.
FIG. 2 is a perspective view showing an animal specimen collection tool according to an embodiment of the present invention.
FIG. 3 is a cross-sectional view showing an animal specimen collection tool according to an embodiment of the present invention.
FIG. 4 is a perspective view showing a combination of a collection portion and a connection adapter according to an embodiment of the present invention.
FIG. 5 is an exploded perspective view showing a tube portion according to an embodiment of the present invention.

### BEST MODE

The present invention having such features may be more clearly described through preferred embodiments according thereto.

Before describing various embodiments of the present invention in detail with reference to the accompanying drawings, it will be appreciated that the application is not limited to the details of configurations and arrangements of elements described in the following detailed description or illustrated in the drawings. The present invention may be implemented and practiced in different embodiments, and may be performed in various ways. Also, it will be appreciated that the expressions and predicates used herein with respect to terms such as the device or element orientation (e.g. "front", "back", "up", "down", "top", "bottom", "left", "right", and "lateral"), etc. are used only to simplify the description of the present invention, and related device or element does not indicate or mean simply a specific orientation. Further, terms such as "first" and "second" are used in this application and the appended claims for purposes of explanation and are not intended to represent or imply any relative importance or spirit.

Accordingly, the embodiments described in the present specification and the configurations shown in the drawings are only the most preferred embodiments of the present invention, and do not represent all the technical ideas of the present invention, it should be understood that there may be various equivalents and variation that may replace these at the time of filing the present application.

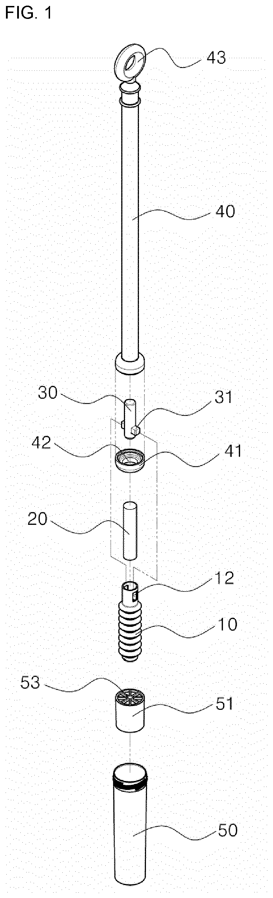

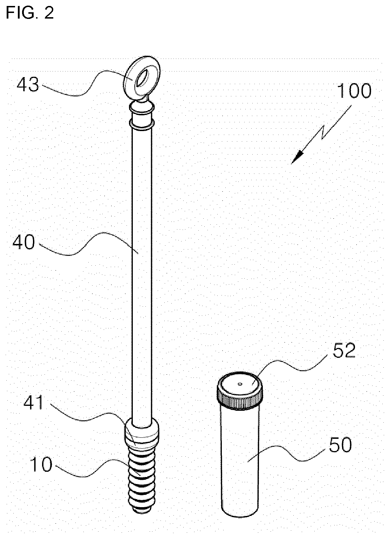

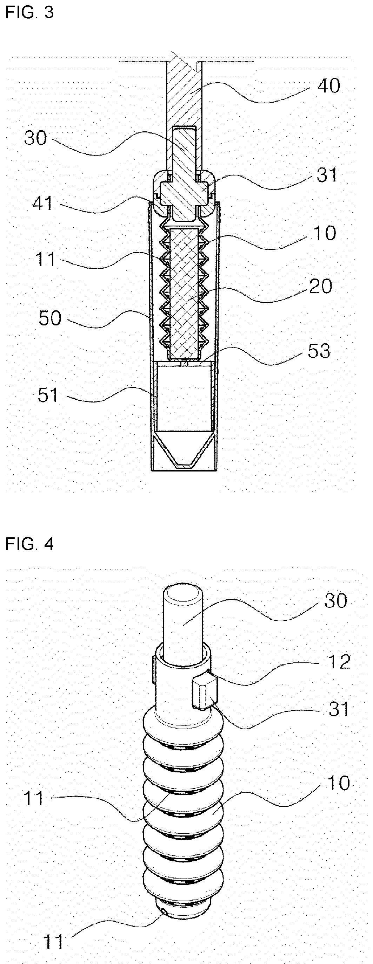

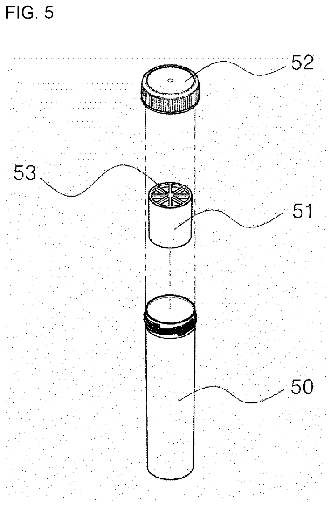

FIG. 1 is an exploded perspective view showing an animal specimen collection tool according to an embodiment of the present invention. FIG. 2 is a perspective view showing an animal specimen collection tool according to an embodiment of the present invention. FIG. 3 is a cross-sectional view showing an animal specimen collection tool according to an embodiment of the present invention. FIG. 4 is a perspective view showing a combination of a collection portion and a connection adapter according to an embodiment of the present invention. FIG. 5 is an exploded perspective view showing a tube portion according to an embodiment of the present invention.

As shown in FIGS. 1 to 5, the animal specimen collection tool of the present invention is a tool 100 directly collecting saliva of animal which is a specimen by putting the tool in the mouth of the animal, and includes a collection portion 10, a sponge 20, a connection adapter 30, a collection stick 40, and a tube portion 50.

As shown in FIGS. 1 to 4, the collection portion 10 is in direct contact with the mouth of the animal to collect the saliva of the animal. The collection portion 10 is in a cylindrical shape, has a hollow inside, one side opened to be communicatively connected to the hollow inside, the opposite side closed, and a plurality of injection holes 11 formed in an outer periphery such that the saliva of animal is introduced.

Here, the collection portion 10 has the outer periphery formed in the shape of a corrugated tube or in a flexible shape such that compressing is easy inside of the tube portion 50, and the plurality of injection holes 11 is formed in an outer surface of the corrugated collection portion 10.

In the opened outer periphery of the collection portion 10, connection holes 12 are formed on both sides of the collection portion for connection of the connection adapter 30, and the collection portion 10 is formed of a silicone or urethane material that is not easily torn in a soft material because animal may bite and chew the collection portion 10.

As shown in FIGS. 1 and 3, the sponge 20 is formed in the hollow inside of the collection portion 10, absorbs the saliva of animal introduced through the input hole 11, and is formed of a hydrophilic material because there should be no problem in storage of DNA/RNA/PROTEIN. At this time, various fruit flavors such as apple flavor are added to the sponge 20 to make the animal generate a large amount of saliva.

Here, the sponge 20 is formed in the same shape as the hollow inner periphery of the collection portion 10. In the present invention, the sponge 20 is formed in a cylindrical shape and combined with the inside of the collection portion 10 through the opened side of the collection portion 10.

As shown in FIGS. 1 and 3 to 4, the connection adapter 30 has a connection protrusion 31 protruding so as to be connected to the connection hole 12 of the collection portion 10, and is fixed to the collection stick 40 while being connected to the collection portion 10, and thus the connection adapter 30 serves to interconnect the collection portion 10 and the collection stick 40.

Here, the connection adapter 30 has the connection protrusion 31 protruding from both sides of the outer periphery to be formed in a "T" shape, is inserted through the opened one side of the collection portion 10 and connected to the collection portion 10 in a structure in which the connection protrusion 31 is coupled to the connection hole 12 inside of the collection portion 10, and is inserted into and fixed to the inside of one side of the collection stick 40 while being connected to the collection portion 10.

In addition, when the connection adapter 30 is inserted into the inside of the collection stick 40, the connection adapter 30 is adhered by ultrasonic waves such that the connection adapter 30 is not separated by strong external force.

As shown in FIGS. 1 to 3, the connection adapter 30 connected to the collection portion 10 is inserted into and fixed to the inside of one side of the collection stick 40, the collection portion 10 is connected to the collection stick 40, and when the collection portion 10 is put into the mouth of the animal, the body of the collection stick 40 is formed to be long such that the collection stick 40 may be used by holding the collection stick 40. At this time, a circular ring portion 43 is formed integrally with the end of the body on the other side of the collection stick 40 so as to be caught by a locking member.

Here, the collection stick 40 is formed of a POM/urethane material such that the collection stick 40 is not broken or cracked when animal bites and chews the collection stick 40, and a fixing cover 41 is installed on one side of the collection stick 40 such that the connection adapter 30 connected to the collection portion 10 is inserted and then fixed. That is, the fixing cover 41 is screwed to one side of the collection stick 40, and when the connection adapter 30 is inserted, the fixing cover 41 is removed from the collection stick 40, the connection adapter 30 is inserted into the collection stick 40, and then the fixing cover 41 is screwed again.

In addition, a through hole 42 is formed in the central portion of the fixing cover 41 such that the collection portion 10 connected to the connection adapter 30 protrudes to the outside, and the connection protrusion 31 is attached to the inner side of the fixing cover 41 such that the connection adapter 30 is fixed without being separated via the through hole 42.

As such, the connection protrusion 31 of the connection adapter 30 is inserted into one side of the collection stick 40 while protruding to the outside through the inner connection hole 12 of the collection portion 10, and the fixing cover 41 is screwed to the collection stick 40, and when the fixing cover 41 is screwed to the collection stick 40 in a state in which the collection portion 10 penetrates the through hole 42 of the fixing cover 41, the connection protrusion 31 of the connection adapter 30 protruding to the outside of the collection portion 10 is attached to the inside of the fixing cover 41 such that the connection adapter 30 and the collection portion 10 are fixed without being separated. In addition, because the fixing cover 41 may be removed, the collection portion 10 and the sponge 20 may be replaced.

As shown in FIGS. 1 to 3 and 5, the tube portion 50 into which the collection portion 10 is inserted compresses the collection portion 10 and the sponge 20 to discharge the saliva of the animal absorbed by the sponge 20 to the outside through the input hole 11 of the collection portion 10, and stores the saliva of the animal discharged from the collection portion 10 therein.

Here, the tube portion 50 is a 50ml conical tube (self standing structure), gamma sterilization is performed to store the saliva of the animal, and a lid portion 52 sealing the inside of the tube portion 50 is screwed to the opened one side of the tube portion 50 such that the saliva of animal is not in contact with the outside. That is, while the lid portion 52 is removed, the collection portion 10 and the sponge 20 are compressed into the tube portion 50 to discharge the saliva of animal into the tube portion 50, and then the collection portion 10 and the sponge 20 are removed and the lid portion 52 is screwed to seal the inside of the tube portion 50.

In addition, a tube adapter 51 in direct contact with the collection portion 10 input into the tube portion 50 to compress the collection portion 10 and the sponge 20 is further formed inside the tube portion 50, and one side surface of the tube adapter 51 is formed in a mesh network 53 structure such that the saliva of the animal discharged by the compression of the collection portion 10 and the sponge 20 penetrates and is stored in the tube portion 50.

In addition, the tube adapter 51 is formed in the same shape as the inner periphery of the tube portion 50, and is formed in a cylindrical shape such that one surface of the mesh net 53 is spaced apart from the bottom surface of the tube portion 50 at a predetermined interval, and thus the saliva of animal stored inside the tube portion 50 does not come into contact with the compressed collection portion 10.

Hereinafter, a collection method of the animal specimen collection tool 100 described above will be described.

First, after connecting the collection portion 10 to the collection stick 40 through the connection adapter 30, when holding the collection stick 40 and inserting the collection portion 10 into the mouth of animal, the animal generates saliva by biting or chewing the collection portion 10 by the fruit flavor, and the saliva generated at this time is introduced through the input hole 11 of the collection portion 10 and is absorbed by the sponge 20.

After putting the collection portion 10 in the mouth of the animal for a certain period of time, the collection portion 10 is removed and immediately inserted into the tube portion 50, the collection portion 10 inserted into the inside of the tube portion 50 is compressed to the tube adapter 51, and the saliva of the animal absorbed in the sponge 20 is discharged into the tube portion 50 through the input hole 11 of the collection portion 10. At this time, the collection portion 10 is easily compressed to the tube adapter 51 because the outer periphery of the collection portion 10 is formed to be flexible.

Then, after the collection portion 10 is separated from the tube portion 50, the lid portion 52 is screwed to the opened one side of the tube portion 50 to seal the inside of the tube portion 50, and in this state the tube portion 50 is transported to a laboratory or the like.

**[Description of Reference numerals]**

| | | | |
|---|---|---|---|
| 10 : | COLLECTION PORTION | 11 : | INJECTION HOLES |
| 12 : | CONNECTION HOLES | 20 : | SPONGE |
| 30 : | CONNECTION ADAPTER | 31 : | CONNECTION PROTRUSION |
| 40 : | COLLECTION STICK | 41 : | FIXING COVER |
| 42 : | THROUGH HOLE | 43 : | CIRCULAR RING PORTION |
| 50 : | TUBE PORTION | 51 : | TUBE ADAPTER |
| 52 : | LID PORTION | 53 : | MESH NETWORK |
| 100 : | ANIMAL SPECIMEN COLLECTION TOOL | | |

## Claims

1. An animal specimen collection tool comprising:
a collection portion in direct contact with a mouth of animal, having a hollow inside to form a plurality of input holes in an outer surface such that saliva of the animal is introduced, one side opened to be communicatively connected to the hollow inside, and a connection hole formed in the opened outer periphery, and formed of a soft, flexible and elastic material;
a sponge formed in the hollow inside of the collection portion, absorbing the saliva of the animal introduced through the input hole, and formed of a hydrophilic material;
a connection adapter having a connection protrusion protruding so as to be connected to the connection hole of the collection portion, and connecting the collection portion and a collection stick;
the collection stick having the connection adapter connected to the collection portion inserted into and fixed to inside of one side; and
a tube portion into which the collection portion is inserted compressing the collection portion and the sponge to discharge the saliva of the animal absorbed by the sponge to the outside through the input hole of the collection portion, and storing the saliva of the animal discharged from the collection portion therein.

2. The animal specimen collection tool of claim 1,
wherein the collection portion is formed in the shape of a corrugated tube so as to be easily compressed inside the tube portion.

3. The animal specimen collection tool of claim 1,
wherein the connection adapter has the connection protrusion protruding from both sides of the outer periphery to be formed in a "T" shape, is inserted through the opened one side of the collection portion to couple the connection protrusion to the connection hole inside the collection portion, and then is inserted into and fixed to the inside of one side of the collection stick.

4. The animal specimen collection tool of claim 3,
wherein a fixing cover is installed on one side of the collection stick such that the connection adapter connected to the collection portion is inserted and then fixed, and a through hole is formed in a central portion of the fixing cover such that the collection portion connected to the connection adapter protrudes to the outside.

5. The animal specimen collection tool of claim 1,
wherein a tube adapter in direct contact with the collection portion input into the tube portion to compress the collection portion and the sponge is further formed inside the tube portion, and one side surface of the tube adapter is formed in a mesh network structure such that the saliva of the animal discharged by the compression of the collection portion and the sponge penetrates and is stored in the tube portion.
